# EUROPEAN PATENT SPECIFICATION

(11) **EP 0 450 352 B1**
(45) Date of publication and mention of the grant of the patent: **31.08.1994**
(21) Application number: 91103796.8
(22) Date of filing: 13.03.1991
(51) Int. Cl.: A61K 9/127, A61K 31/425, A61K 37/02

(54) **Liposome formulations of immunomodulating drugs for the topical and aerosol administrations**
Liposomformulierungen immunmodulierender Wirkstoffe für topische und Aerosol-Anwendungen
Formulations de liposomes d'agents immunomodulateurs pour l'administration topique et l'administration comme aérosol

(30) Priority: 30.03.1990 IT 1988990
(43) Date of publication of application: 09.10.1991
(73) Proprietor: POLI INDUSTRIA CHIMICA S.p.A., 20141 Milano (IT)
(72) Inventor: Poli, Stefano, I-20141 Milano (IT); Moro, Luigi, I-20141 Milano (IT); Natali, Alberto, I-20141 Milano (IT); Coppi, Germano, I-20141 Milano (IT)
(74) Representative: Minoja, Fabrizio

(56) References cited:
- EP-A- 0 177 223
- EP-A- 0 225 130
- EP-A- 0 253 619
- EP-A- 0 267 050
- EP-A- 0 276 752
- EP-A- 0 335 726
- WO-A-85/00751
- WO-A-88/00824
- ORGANIC-CHEMICAL DRUGS AND THEIR SYNONYMS, 6th edition, vol. I, 1987, M.NEGWER, Akademie-Verlag, Berlin, pages 33,107

## Description

The present invention relates to liposome formulations suitable for the administration of immunomodulating drugs.

Liposomes are spherules of submicron size consisting of concentric layers of phospholipid material, which are alternated to discrete and isolated aqueous spaces.

The composition of these liposome vesicles, the main components of which are substances widely present in the biologic membranes, affects and determines, besides the chemico-physical stability of the originated structures, also the pharmacokinetic profile and therefore the carrier capability thereof.

Liposomes have been known for a long time as possible carriers for pharmacologically active substances and the use thereof in the chemotherapeutic field is subject-matter of a number of patents and scientific literature. Liposomes are used in therapy mainly in the parenteral formulations, whereas the use of liposomes for the oral and topical administration is less frequent. The main feature of this kind of formulation is an improvement in the therapeutic index, due to a reduction in toxicity, by means of the inclusion of the active ingredient, and to an increase in drug efficacy thanks to a helped transport through the cell membrane.

The problem with parenteral therapy and the main reason for which the efficacy of this kind of administration can be reduced is constituted by the stimulation of the reticuloendothelial system, and particularly of the circulating macrophages: in fact, the latter are responsible for the capture of the particulate carriers and for the degradation of their phospholipid structure mainly due to lysosomial enzymes.

Nevertheless, this drawback can advantageously be utilized in case of an immunomodulating therapy supported with a series of topical applications of the active ingredient carried by liposomes. It is therefore evident that, in case of an administration of a specifically immunostimulating drug by means of a liposome carrier, a synergism can take place.

Different kinds of and different preparation methods of liposomes are known: for instance, EP-A-0253619 discloses single bilayered liposomes; EP-A-0225130 discloses liposome compositions characterized by an aqueous disperse medium having an osmotic pressure higher than the osmotic pressure of the solution used for entrapping the drug in liposomes; EP-A-0177223 discloses multi-lamellar lipid vesicles encapsulating different kinds of active principles, including immunomodulators in general.

The present invention relates to a formulation of selected immunomodulating drugs in a liposome carrier and its use in therapy by means of a topical administration.

The formulation consists of a liposome suspension, which is obtained according to well-known methods and can be used as such or after addition of a viscosity modulator selected from the group consisting of either the synthetic thickening agents, such as cellulose, acrylic or vinyl derivatives, or the semisynthetic or natural ones, such as alginates or pectins. Particularly preferred is carboxyvinyl polymer (Carbomer®), in concentrations ranging from 0.1 to 3% w/v, most preferably 1%, even subsequently salified to pH between 4 and 7.5, preferably between 4 and 5.

Further advantages arise from the use of a liposome formulation for the topical or localized administration of an immunomodulating drug, since :
a) the active ingredient is more protected against any oxidation caused by atmospheric oxygen;
b) the cells in the application site are more protected against a massive contact with local high concentrations of active ingredient;
c) a combination of poorly compatible substances to administer can be accomplished, keeping only a few of them included in the liposome structure and not in contact with the other ones outside;
d) the efficacy time of the drug can be varied so as to induce a long-lasting effect of the drug;
e) the inclusion in structures having an individual mass can promote the increase of macrophage local density, i.e. acting as an active targeting element.

The liposome formulation can be a liposome suspension or a gel obtained by adding the suspension with substances which can increase the preparation viscosity. The liposome suspension consists of :
1) one or more polar phospholipid or lipid substances acting as a support of the liposome carrier, due to their natural ability to put on a bilayer arrangement in case of hydration;
2) one or more substances which can modify the transition temperature typical of each phospholipid, selected from the group consisting of aliphatic or aromatic alcohols, sterols (cholesterol), mixtures and/or esters thereof;
3) optionally, one or more substances carrying ionizable groups which are partially or totally dissociated at the pH of the preparation, thus acting as charge carriers which can give the liposome a negative or positive net superficial charge. Dicethylphosphate, phosphatidic acid, phosphatidylserine, phosphatidylethanolamine, stearylamine, cardiolipin are examples of this class of substances;
4) optionally one or more antioxidant agents, which can counteract the tendency of lipid substances to form peroxides, depending on the insaturations of fatty acids which constitute them. Examples of this class of substances are tocopherols, esters thereof, butylhydroxyanisole, butylhydroxytoluene, β-carotene;
5) optionally, one or more chelating agents complexing or fixing, through cohordination bonds, multivalent metal ions which can act either as aggregation centres for liposome structures or as catalysts for oxidative degradation reactions ;
6) a solvent phase which can dissolve liposome structures without impairing their integrity and solubilize the remaining components of the formulation;
7) one or more pharmacologically active substances selected from 3-L-pyroglutamyl-L-thiazolidine-4-carboxylic acid (PGT/1A, EP-A-0276752), thymopentine and arginine thiazolidinecarboxylate, respectively in concentrations from 0.01 to 20 w/v, from 0.05 to 20% w/v and from 0.1 to 10% w/v. which, depending on the partition coefficient thereof, can be present in the liposome lipid phase or in the aqueous phase which acts as a partition element between them;
8) one or more substances which can give the formulation the specific features of resistance against bacterial contamination which are appropriate to a non exclusively single-dose pharmaceutical product. Methyl, ethyl, propyl and butyl hydroxybenzoates, benzoic acid and the salts thereof, sorbic acid and the salts thereof, dehydroacetic acid and the salts thereof are some examples of such substances;
9) other optional excipients such as wetting agents, perfumes, essences, dyes and the like.

One test for the immunostimulating activity after intraperitoneal administration of liposome preparations of the immunostimulating compound 3-L-pyroglutamyl-L-thiazolidine-4-carboxylic acid (PGT/1A) on peritoneal macrophages of prednisolone-immunodepressed rat, proved that the liposome formulation according to the invention acts by activating chemotaxis and superoxide anion production in a higher manner than administering a PGT/1A aqueous solution.

The following examples further explain the invention.

### EXAMPLE 1

180 g lecitin, 52 g cholesterol, 5 g butylhydroxyanisole, 25 g trometamol and 50 g PGT/1A (3-L-pyroglutamyl-L-thiazolidine-4-carboxylic acid) are dissolved in 250 ml ethyl alcohol.

After dissolution, 4400 ml of a 0,1M glycine solution are added under vigorous stirring. Alcohol is evaporated off with heating, to obtain a liposome suspension which is easily filtered through a 1 µm porosity membrane before sharing it in vials for aerosol administration.

### EXAMPLE 2

1000 ml of the liposome suspension of the example 1 are gelated by addition of Carbomer® to a 1% w/v final concentration, then salified with sodium hydroxide. A gel with a suitable consistency for vaginal administration is obtained.

### EXAMPLE 3

36 g lecitin, 10 g cholesterol, 1 g Vitamin E acetate and 10 g thymopentin are dissolved in 50 ml ethyl alcohol. One liter of a 0.25% w/v sodium edetate aqueous solution is prepared separately and warmed to 65°C. After mixing the two solutions under strong stirring and removing alcohol, a liposome suspension is obtained which passes through a 3 µm porosity membrane. The filtered suspension is shared in heat-sealed vials for aerosol administration.

## Claims

1. Liposome formulations suitable for drug administration, characterized in that they include:
(a) a phospholipid, a mixture thereof or natural polar lipids;
(b) an alifatic or aromatic alcohol, sterols, mixtures and/or esters thereof;
(c) a mainly aqueous dispersing solvent;
(d) optionally antioxidant and chelating agents and optionally further excipients;
(e) optionally a charge-bearing substance;
(f) one or more drugs selected from 3-L-pyroglutamyl-L-thiazolidine-4-carboxylic acid, thymopentin, arginine thiazolidinecarboxylate, respectively in concentrations from 0.01 to 20% w/v, from 0.05 to 20% w/v and from 0.1 to 10% w/v.

2. Liposome formulations according to claim 1, suitable for topic and aerosol administrations.

3. Liposome formulations according to claims 1-2 in form of a gel obtainable by adding a thickening agent.

4. Liposome formulations according to claim 3, wherein the liposome suspension is thickened by addition of a carboxyvinyl polymer in a concentration from 0.1 to 3%, preferably about 1.1% w/v.

5. Liposome formulations according to claim 1 in form of lavage and/or any other carrier suitable for vaginal administration.

## Patentansprüche

1. Liposom-Formulierungen, die geeignet sind für die Arzneimittel-Verabreichung, dadurch gekennzeichnet, daß sie enthalten:
a) ein Phospholipid, eine Mischung davon oder natürliche polare Lipide;
b) einen aliphatischen oder aromatischen Alkohol, Sterine, Mischungen und/oder Ester davon;
c) ein hauptsächlich wäßriges Dispergier-Lösungsmittel;
d) gegebenenfalls Antioxidantien und Chelatbildner und gegebenenfalls weitere Exzipienten;
e) gegebenenfalls eine Ladungen tragende Substanz; und
f) ein oder mehr Arzneimittel, ausgewählt aus der Gruppe 3-L-Pyroglutamyl-L-thiazolidin-4-carbonsäure, Thymopentin, Argininthiazolidincarboxylat, jeweils in Konzentrationen von 0,01 bis 20 Gew./Vol.-%, 0,05 bis 20 Gew./Vol.-% bzw. 0,1 bis 10 Gew./Vol.-%.

2. Liposom-Formulierungen nach Anspruch 1, die geeignet sind für topische und Aerosol-Verabreichungen.

3. Liposom-Formulierungen nach den Ansprüchen 1 bis 2 in Form eines Gels, das durch Zugabe eines Verdickungsmittels erhältlich ist.

4. Liposom-Formulierungen nach Anspruch 3, worin die Liposom-Suspension durch Zugabe eines Carboxyvinylpolymers in einer Konzentration von 0,1 bis 3 Gew./Vol.-%, vorzugsweise von etwa 1,1 Gew./Vol.-%, verdickt ist.

5. Liposom-Formulierungen nach Anspruch 1 in Form einer Spülung und/oder irgendeines anderes Trägers, der (die) für die vaginale Verabreichung geeignet ist.

## Revendications

1. Formulations aux liposomes convenant pour l'administration de médicaments, caractérisées en ce qu'elles comprennent :
(a) un phospholipide, un mélange de phospholipides ou des lipides polaires naturels ;
(b) un alcool aliphatique ou aromatique, des stérols, des mélanges et/ou esters de ceux-ci ;
(c) un solvant de dispersion principalement aqueux ;
(d) facultativement, un antioxydant et des agents chélateurs, et facultativement des excipients supplémentaires ;
(e) facultativement, une substance porteuse de charges ;
(f) un ou plusieurs médicaments choisis parmi l'acide 3-L-pyroglutamyl-L-thiazolidine-4-carboxylique, la thymopentine, le thiazolidinecarboxylate d'arginine, respectivement en des concentrations de 0,01 à 20 % en poids/volume, de 0,05 à 20 % en poids/volume et de 0,1 à 10 % en poids/volume.

2. Formulations aux liposomes selon la revendication 1, convenant pour des administrations locales et en aérosol.

3. Formulations aux liposomes selon les revendications 1 et 2, sous forme d'un gel pouvant être obtenu par addition d'un épaississant.

4. Formulations aux liposomes selon la revendication 3, dans lesquelles la suspension de liposomes est épaissie par addition d'un polymère carboxyvinylique en une concentration de 0,1 à 3%_{,} de préférence d'environ 1,1 % en poids/volume.

5. Formulations aux liposomes selon la revendication 1, sous forme d'un liquide de lavage et/ou de tout autre véhicule convenant pour une administration vaginale.
